# EUROPEAN PATENT SPECIFICATION

(11) **EP 3 081 249 B1**
(45) Date of publication and mention of the grant of the patent: **30.12.2020**
(21) Application number: 16156472.9
(22) Date of filing: 20.11.2013
(51) Int. Cl.: A61K 38/28, A61K 9/00, A61M 5/32, A61M 5/20, A61M 5/14, A61M 25/00, A61M 25/06, A61M 5/30, A61M 5/142, A61M 5/42, A61M 5/158, A61M 25/02, C12N 9/64, C07K 14/535, A61K 38/19, A61K 38/26, A61M 5/31

(54) **DRUG DELIVERY DEVICE**
ARZNEIMITTELABGABEVORRICHTUNG
DISPOSITIF D'ADMINISTRATION DE MÉDICAMENTS

(30) Priority: 21.11.2012 US 201261729303 P; 07.03.2013 US 201361774567 P
(43) Date of publication of application: 19.10.2016
(62) Document number(s) of the earlier application(s) in accordance with Art. 76 EPC: 13802787.5 / 2 922 590
(73) Proprietor: Amgen Inc., Thousand Oaks, California 91320-1799 (US)
(72) Inventor: MEHTA, Dhairya, South San Francisco, CA 94080 (US); TOY, Stephanie, Moorpark, CA 93021 (US); RING, Lawrence, Valencia, CA 91355 (US); TAMTORO, Ferry, Newbury Park, CA 91320 (US); CAIRNS, Alexander Stuart, Santa Monica, CA 90404 (US); GIBSON, Scott Robert, Grenada Hills, CA 91344 (US)
(74) Representative: Grünecker Patent- und Rechtsanwälte PartG mbB

(56) References cited:
- WO-A1-03/022337
- WO-A1-2006/032689
- WO-A1-2010/080715
- WO-A1-2011/156373
- WO-A2-2009/158655

## Description

### Background

This patent is directed to a drug delivery device, and, in particular, to a drug delivery device for use with a blunt cannula or rigid needle.

Drug delivery devices can administer a bolus at high flow rates, see for instance document WO 2011/156373 or document WO 2006/032689 or document WO 2009/158655 or document WO 03/022337 or WO 2010/080715. Such drug delivery devices include, but are not limited to, autoinjectors, infusion pumps and microinfusers. A microinfuser can be an on-body pump that may be worn continuously. At such high flow rates, the flow of a drug can become interrupted when a buildup of pressure occurs at the tip of the needle or cannula used to administer the bolus. The buildup of pressure may occur when, for example, the opening at the end of the needle or cannula is occluded or blocked. The interruption of the flow through the needle or cannula can have negative effects, such as preventing delivery of the correct amount of drug product or preventing delivery of the drug product at the desired rate (i.e., a lower rate must be used).

### Summary

The present invention relates to a wearable drug delivery device as defined in claims 1 and 4; preferred embodiments of the drug delivery device are defined in the dependent claims. According to an aspect of the present disclosure, a drug delivery device includes a blunt cannula and a reservoir. The blunt cannula has a cylindrical wall that defines an axial passage between a first end and a second end of the blunt cannula. The wall has at least a first tapered region at the first end to define an opening in fluid communication with the axial passage and adapted at the first end to resist interruption of fluid flow through the axial passage and out of the first end of the blunt cannula. The reservoir is connected to the second end of the blunt cannula.

According to another aspect of the present disclosure, a drug delivery device includes a blunt cannula and a reservoir. The blunt cannula has a cylindrical wall that defines an axial passage between a closed first end and a second end of the blunt cannula. The wall has a first tapered region at the first end with at least one side opening in fluid communication with the axial passage adapted to resist interruption of fluid flow through the axial passage and out of the first end of the blunt cannula. The reservoir is connected to the second end of the blunt cannula.

According to a further aspect of the present disclosure, a drug delivery device includes a blunt cannula, a vibration generator, and a reservoir. The blunt cannula has a cylindrical wall that defines an axial passage between a first end and a second end of the blunt cannula. The wall has in a first tapered region at the first end to define an opening in fluid communication with the axial passage. The vibration generator is coupled to the blunt cannula, the generator being actuated to resist interruption of fluid flow through the axial passage and out of the first end of the blunt cannula. The reservoir connected to the second end of the blunt cannula.

According to a still further aspect of the present disclosure, a drug delivery device includes a rigid needle and a reservoir. The rigid needle has a cylindrical wall that defines an axial passage between a first end and a second end of the rigid needle. The wall has an opening at the first end in fluid communication with the axial passage and adapted at the first end to resist interruption of fluid flow through the axial passage and out of the first end of the rigid needle. The adaptation at the first end includes at least one of a pattern of openings disposed about the opening in the first tapered region, and at least one external recessed region recessed toward the axial passage relative to adjoining surface regions. The reservoir is connected to the second end of the rigid needle.

According to yet another aspect of the present disclosure, a drug delivery device includes a rigid needle, a vibration generator and a reservoir. The rigid needle has a cylindrical wall that defines an axial passage between a first end and a second end of the rigid needle. The wall has an opening at the first end in fluid communication with the axial passage. The vibration generator is coupled to the rigid needle, the generator being actuated to resist interruption of fluid flow through the axial passage and out of the first end of the rigid needle. The reservoir is connected to the second end of the rigid needle.

### Brief Description of the Drawings

It is believed that the disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.
Fig. 1 is a schematic view of a drug delivery device according to the present disclosure, including a blunt cannula adapted to resist interruption of fluid flow through the cannula;
Fig. 2 is a perspective view of an embodiment of a blunt cannula according to the present disclosure that may be used with a drug delivery device, such as is illustrated in Fig. 1, with at least one pair of side ports;
Fig. 3 is a side view of the blunt cannula of Fig. 2;
Fig. 4 is an enlarged side view of a first end of the blunt cannula of Fig. 2;
Fig. 5 is a cross-sectional view of the blunt cannula of Fig. 4 taken along line 5-5;
Fig. 6 is an enlarged side view of a first end of another embodiment of a blunt cannula according to the present disclosure with at least one pair of side ports having a different shape than those of Figs. 2-5;
Fig. 7 is an enlarged side view of a first end of another embodiment of a blunt cannula according to the present disclosure with a single bevel;
Fig. 8 is an enlarged side view of a first end of another embodiment of a blunt cannula according to the present disclosure with a single offset bevel;
Fig. 9 is an enlarged side view of a first end of another embodiment of a blunt cannula according to the present disclosure with a pair of positive, intersecting bevels;
Fig. 10 is an enlarged side view of a first end of another embodiment of a blunt cannula according to the present disclosure with a pair of negative, intersecting bevels;
Fig. 11 is an enlarged side view of a first end of another embodiment of a blunt cannula according to the present disclosure with a first pattern of recesses disposed about an axial opening;
Fig. 12 is an enlarged side view of a first end of another embodiment of a blunt cannula according to the present disclosure with a second pattern of recesses disposed about an axial opening;
Fig. 13 is an enlarged side view of a first end of another embodiment of a blunt cannula according to the present disclosure with a pattern of transverse openings disposed about a capped first end;
Fig. 14 is an enlarged side view of a first end of another embodiment of a blunt cannula according to the present disclosure with having recessed surface regions on an external surface of the cannula defined by a pattern of ribs formed on the external surface;
Fig. 15 is an enlarged side view of a first end of another embodiment of a blunt cannula according to the present disclosure with having recessed surface regions on an external surface of the cannula defined by a pattern of grooves formed in the external surface;
Fig. 16 is a schematic view of a drug delivery device according to the present disclosure, including a blunt cannula (which may or may not have features of the blunt cannulas illustrated in Figs. 2-15) and a vibration generator;
Fig. 17 is an enlarged schematic view of a drug delivery device according to the present disclosure and a region of skin to which the drug delivery device is applied;
Fig. 18 is an enlarged plan view of the region of skin illustrated in Fig. 17, with a point of introduction or insertion of the cannula or needle illustrated relative to a region of skin to which the drug delivery device is applied with adhesive;
Fig. 19 is an enlarged schematic view of the drug delivery device of Fig. 17 in an intermediate state or configuration as the cannula or needle is introduced into the skin; and
Fig. 20 is an enlarged schematic view of the drug delivery device of Fig. 17 in a final state or configuration with the cannula or needle fully introduced or inserted into the skin.

### Detailed Description of Various Embodiments

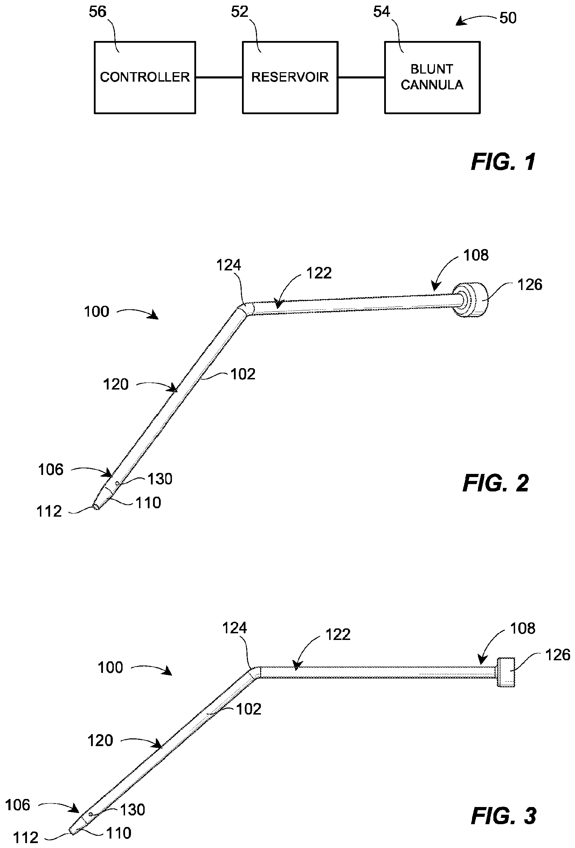

Fig. 1 is a schematic diagram of a drug delivery device 50 according to the present disclosure, which drug delivery device 50 may be in the form of an autoinjector, infuser or microinfuser, for example. For the purpose of clarification, reference to one of these devices does not preclude use of other drug delivery devices. The drug delivery device 50 includes a reservoir 52 and a blunt cannula 54. The blunt cannula 54 has a first end and a second end, the reservoir 52 connected to the second end of the blunt cannula 54 and the first end used for subcutaneous delivery of a drug product from the reservoir to a patient. The reservoir can be any primary container, e.g. a prefilled syringe or a cartridge. The drug delivery device 50 also includes a controller 56 that is operatively coupled to the reservoir 52, and may even preferably be formed integrally with the reservoir 52. The controller 56 may include a drive, which may be mechanical, electromechanical, or electrical, that is operatively coupled to the reservoir 52 to force fluid from the reservoir 52 through the blunt cannula 54. For example, where the reservoir 52 is defined by a barrel and a plunger disposed within the barrel, the drive may incorporate a mechanical element that advances the plunger along the barrel to force a drug product from the reservoir 52. The controller 56 may also include a microprocessor which is operatively coupled to the drive to cause the drive to actuate. In some embodiments, the controller 56 may simply be a plunger rod in a syringe.

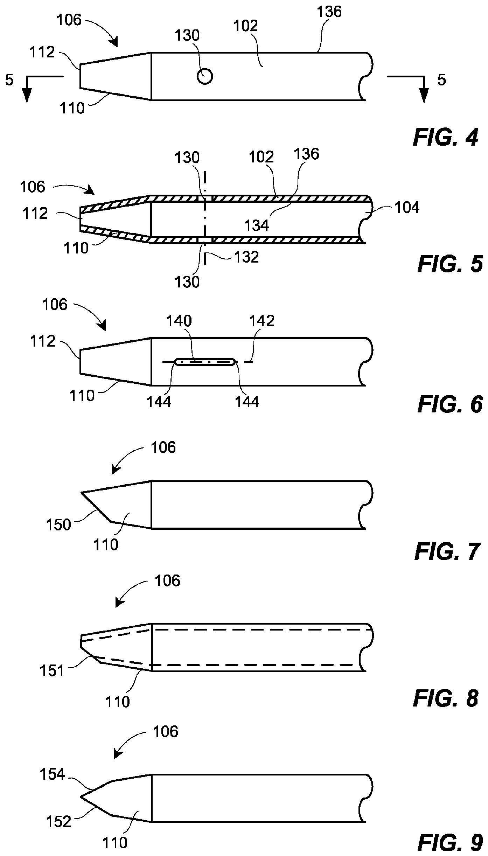

Figs. 2-14 illustrate a variety of blunt cannulas according to the present disclosure. In particular, Figs. 2-5 illustrate one embodiment of a blunt cannula, the details of which are discussed so that the structure and function of the remaining embodiments illustrated in Figs. 6-15 might be appreciated without repeating the details of structure and function common with the embodiment of Figs. 2-5 for each of these additional embodiments. Instead, only the details of structure and function that differentiate the embodiments illustrated in Figs. 6-15 from the embodiment of Figs. 2-5 will be discussed in relation to Figs. 6-15.

Fig. 2 illustrates a blunt cannula 100 having a cylindrical wall 102 that defines an axial passage 104 best seen in Fig. 5. The axial passage 104 extends between a first end 106 and a second end 108 of the blunt cannula 100. The wall 102 has at least a first tapered region 110 at the first end 106 to define an opening 112 in fluid communication with an axial passage 104.

As regards the specific embodiment of the blunt cannula 100 illustrated in Fig. 2, it will be recognized that the cannula 100 includes a first segment 120 and a second segment 122 that are connected by a joint or transition 124. The first segment 120 and the second segment 122 are disposed at an angle to each other, as best seen in Fig. 3. According to the illustrated embodiment, the first segment 120 and the second segment 122 are disposed at an obtuse angle relative to each other. Even though the segments 120, 122 are disposed at an angle to each other, the passage 104 may still be referred to as the axial passage. Moreover, while Figs. 1 and 2 show a cannula with segments disposed at an angle to each other, there may be additional embodiments where the segments are not at an angle to each other and the cannula could in fact be a single straight segment.

In some embodiments, a hub 126 is disposed at the second end 108 of the cannula 100, while in other embodiments a hub may not be present. The hub 126 may surround a needle or other connector used to connect the cannula 100 with a reservoir such that the cannula 100 and the reservoir are in fluid communication with each other (e.g., by having the needle pierce a rubber septum or the like). While the structure has been explained in regard to the illustrated embodiment, the cannula according to the present disclosure is not so limited, and variations may exist to the cannula illustrated in Figs. 2 and 3.

The first end 106 also is adapted to resist interruption of fluid flow through the axial passage 104 and out of the first end 106 of the blunt cannula 100. As will be discussed in regards to the remaining embodiments, the manner in which the first end 106 is adapted to resist interruption of fluid flow may vary. In fact, while a variety of adaptations are discussed individually in regards to Fig. 2-15, it will be appreciated that it is possible to combine the specific embodiments discussed in each of the illustrations of Figs. 2-15 with each other. For example, the embodiments of Figs. 2-5 or Fig. 6 may be combined with the embodiment of Fig. 7. Still further combinations will be apparent to those skilled in the art, and discussed below.

As to the adaptation illustrated in Figs. 2-5, this embodiment has at least one pair of side ports 130 formed in the wall 102 of the blunt cannula 100 at the first end 106, although the present disclosure would also encompass an embodiment that includes at least one side port (e.g., only one side port). The at least one pair of side ports 130 illustrated in Figs. 2-5 are in the form of circular openings formed in the wall 102 of the blunt cannula 100 at the first end 106. Further, as best seen in Fig. 5, the circular openings formed in the wall 102 of the blunt cannula 100 are aligned with each other across the axial passage 104 (i.e., the circular openings lie along a transverse axis 132), however such alignment is not necessary. For example, the side ports 130 may be at different distances from the first end 106 of the cannula 100.

In regard to the two side ports 130, it will be recognized that the wall 102 of the blunt cannula 100 has an internal surface 134 and an external surface 136. The side ports 130 have a first opening on the internal surface 134 and a second opening on the external surface 136, and a passage connecting the first and second openings. Consequently, the side ports 130 depend through the wall 102 of the cannula 100.

As will be further recognized, the side ports 130 of the embodiment illustrated in Figs. 2-5 are formed in the first end 106 of the blunt cannula 100 distal from the first tapered region 110 that defines the opening 112. In other embodiments, the side ports 130 may be disposed within the first tapered region 110. In still other embodiments, the side ports 130 are disposed further from the first tapered region 110.

According to a specific embodiment of the present disclosure, the side ports may have a diameter of 0.006 inches (0.15 mm), and may be disposed 0.06 inches (1.5 mm) from the opening 112 for a 24 gauge cannula. In an alternative embodiment, the side ports may have a diameter of 0.006 inches (0.15 mm), and may be disposed 0.08 inches (2 mm) from the opening 112 for a 24 gauge cannula. In yet other embodiments the diameter and the distance from the opening 112 may be different than the values set forth immediately above. The ports may be formed using a drill, for example.

Fig. 6 illustrates a related embodiment of the present disclosure wherein at least one pair of side ports is formed in the wall 102 of the blunt cannula 100. However, unlike the embodiment illustrated in Figs. 2-5, the embodiment of the side port illustrated in Fig. 6 is in the form of an elongated slit or an elongated slot 140 (wherein the slit has a narrow width relative to the slot). Specifically, the elongated slit 140 may lie along an axis 142 that runs parallel to the axial passage 104. Further, the elongated slit 140 may have first and second rounded ends 144. As was the case relative to the side ports 130, the slits 140 may have in a first opening in the internal surface 134 and a second opening in the external surface 136 of the blunt cannula 100, and a passage connecting the first and second openings. The slit may be formed using laser cutting tools, for example.

Figs. 7-9 illustrate a different adaptation for resisting the interruption of fluid flow through the axial passage 104. In particular, the embodiments illustrated in Figs. 7-9 involve the formation of at least one bevel in the first end 106 of the cannula 100. In particular, the at least one bevel is formed in the first tapered region 110 that defines the opening 112. As a consequence, whereas the opening 112 illustrated in the embodiments of Figs. 2-6 may be circular in cross-section, the opening 112 of the embodiments illustrated in Figs. 7-10 may have at least one oblong or oval face, and may in fact have oval faces in multiple planes inclined relative to the axial passage 104.

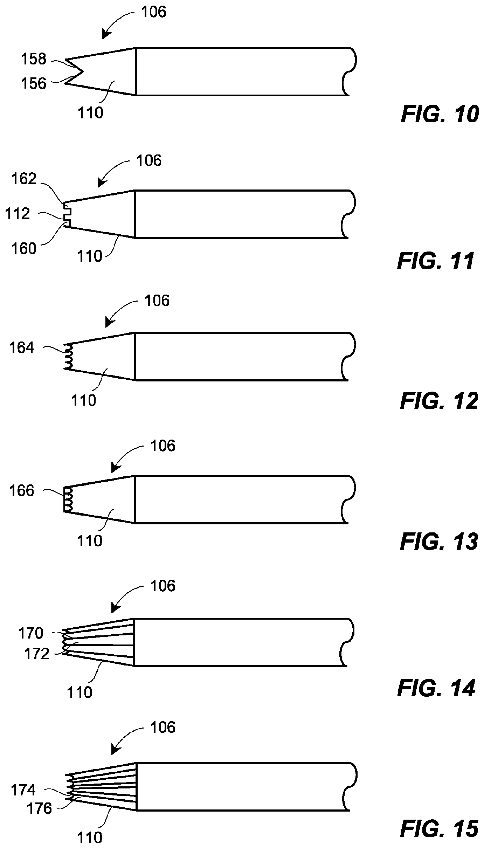

In particular, the embodiments illustrated in Figs. 7 and 8 include only a single bevel 150, 151, although the embodiment of Fig. 7 has the bevel across the entire tapered region 110 while the embodiment of Fig. 8 has the bevel across only a portion of the tapered region 110 (which may make the end of the embodiment of Fig. 8 more resistant to buckling or bending as a consequent). The bevel 150, 151 may be referred to as a positive bevel herein. In a similar vein, Fig. 9 illustrates the use of two positive bevels 152, 154 intersecting each other. However, it is also possible to use an inverted or negative bevel, as is done in Fig. 10 wherein an inverted double bevel 156, 158 is used. As mentioned previously, any one of the embodiments illustrated in Figs. 7-10 may be used with any one of the embodiments illustrated in Figs. 2-6 in so far as the side ports do not interfere with the function of the bevel or bevels formed in the tapered region 110.

As illustrated, the bevel may be inclined at 45 degrees. However, it will be recognized that other angles of bevel are possible. It will also be recognized that shallow angles resist buckling of the blunt cannula when the cannula is inserted into or through the skin of the patient.

Figs. 11-13 illustrate further adaptations for resisting the interruption of fluid flow through the axial passage 104. In particular, the embodiments illustrated in Figs. 11-13 involve a pattern of openings disposed about the opening 112 in the first tapered region 110. The pattern of openings may be regular and periodic as illustrated, or the pattern of openings may be irregular (i.e., the spacing may not be equal relative to the openings and the intervening wall sections).

For example, the embodiment of Fig. 11 includes a regular and periodic pattern of openings 160 and wall sections 162. The resulting pattern may be referred to as castellated. Similarly, a second pattern of openings 164 is illustrated in Fig. 12, which openings 164 may be defined as recesses in the tapered region 110 formed by a series of grooves transverse to the axial passage 104. In fact, a still further pattern of openings 166 is illustrated in Fig. 13, wherein the first end 106 is capped such that fluid flow is only possible transverse to the axial passage 104 through the openings 166. The openings 166 may be referred to as windows.

As was the case relative to the embodiments illustrated in Figs. 7-10, the embodiments illustrated in Figs. 11-13 may be used in combination with any of the embodiments illustrated in Figs. 2-5. Alternatively, the embodiments illustrated in Figs. 11-13 may be used in combination with any of the embodiments illustrated in Figs. 7-10. Still further, the embodiments illustrated in Figs. 11-13 may be used in combination with the embodiments of Figs. 2-6 and 7-10.

Figs 14 and 15 illustrate still further adaptations for minimizing or reducing the interruption of fluid flow through the axial passage 104 as well as resisting buckling of the cannula 100. In particular, the wall 102 of the blunt cannula 100 may have at least one external recessed region formed thereon, the recess being recessed towards the axial passage 104 relative to adjoining surface regions. This at least one external recessed region may be in a pattern that is both regular and periodic, or the pattern of openings may be irregular (i.e., the spacing may not be equal relative to the first tapered region 110).

According to the embodiment illustrated in Fig. 14, the at least one recessed region 170 may be defined by a pattern of ribs 172 formed on the external surface 136. Alternatively, the least one recessed region 174 in Fig. 15 may be defined by a pattern of grooves 176 formed in the external surface 136. It may also be possible to define the least one recessed region using the combination of a pattern of ribs and a pattern of grooves in combination. It is preferred that the ribs or grooves terminate before the portion of the cannula 100 that depends from the insertion site so as to limit the possibility of leakage from the site. It is also preferred that the ribs or grooves be used with at least one side port or opening along the perimeter of the opening 112, although the ribs or grooves may be used without providing a side port or other opening (e.g., with a beveled end).

As was the case relative to the embodiments illustrated above, the embodiments illustrated in Figs. 14 and 15 may be used in combination any one of the embodiments illustrated in Figs. 2-13. It is the case that the embodiments of Figs. 14 and 15 may be most useful with an embodiment where a radial flow path is provided, such as with the provision of a side port, tip groove, or the like. Alternatively, the embodiments illustrated in Figs. 14 and 15 may be used in combination with any of the embodiments illustrated any one or more of the groups of adaptations in Figs. 2-6, 7-10 or 11-13. Consequently, the embodiments illustrated in Figs. 14 and 15 may be used in combination with embodiments section from each of the groups illustrated in Figs. 2-6, 7-10, and 11-13.

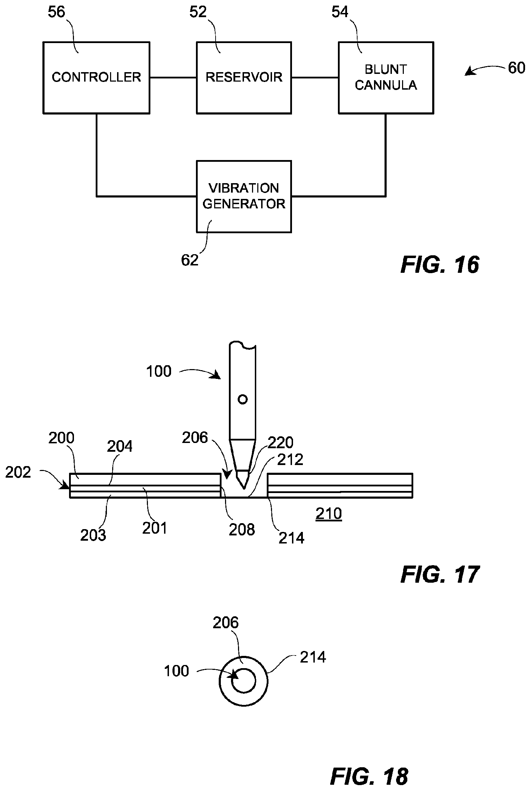

A still further adaptation according to the present disclosure is illustrated in Fig. 16. According to this embodiment, in addition to the reservoir 52, cannula 54, and controller 56, the drug delivery device 60 includes a vibration generator 62. The operation of the vibration generator 62 may be controlled by the controller 56 to which it is operatively coupled. In addition, the output of the vibration generator 62 may be operatively coupled to the cannula 54. The vibration generator may be in the form of a motor having a shaft with an eccentric weight attached to the shaft. Alternatively, the vibration generator may be in the form of a piezoelectric vibrator. Still further alternative embodiments are possible. Actuation of the vibration generator 62 may dislodge an obstruction or blockage from the end of the cannula 54, move the tip of the cannula 54 away from the occluding structure (e.g., tissue), or create a pocket of micro-fractured tissue increasing the surface area for lower pressure threshold, thereby permitting flow to resume

As was the case with the adaptations recited above, the adaptation illustrated in Fig. 16 may be used with any one or more of the adaptations illustrated in Figs. 2-15.

In addition, while the previous embodiments have been discussed in regard to a blunt cannula, certain of the above-mentioned adaptations may also be used with a rigid needle as well, which needle may be made of metal and have a point defined by one or more bevels made at a first end of the needle. The rigid needle may have a cylindrical wall that defines a passage between the first end and a second end, and the first end may have an opening that is in fluid communication with the axial passage. Moreover a reservoir may be connected to the second end of the rigid needle.

In particular, the adaptations according to Figs. 11, 12 and 14-16 may be used with the rigid needle.

As will be recognized, the devices according to the present disclosure may have one or more advantages relative to conventional technology, any one or more of which may be present in a particular embodiment in accordance with the features of the present disclosure included in that embodiment. In particular, each of the embodiments illustrated in Figs. 2-15 provides alternative flow paths for the drug product should the opening in the first end of the blunt cannula be obstructed or blocked, thereby limiting the possibility of the interruption of the flow through the cannula. The embodiments also increase the surface area exposed, lowering the pressure threshold required to displace tissue. The embodiment illustrated in Fig. 16 uses a different mechanism by which to limit the interruption of flow through the cannula, wherein the vibrations may dislodge an obstruction or blockage from the end of the cannula, move the cannula tip away from the occluding structure (e.g., tissue), or create a pocket of micro-fractured tissue increasing the surface area for lower pressure threshold, thereby permitting flow to resume. Other advantages not specifically listed herein may also be recognized as well.

Figs. 17-20 illustrate how the systematic approach to lowering the pressure required to pass fluid through a cannula or needle provided by the embodiments according to the present disclosure may operate in a particular application of this technology. In particular, this discussion is in relation to a wearable drug delivery device, which is one that is attached to the patient's skin and includes its own mechanism(s) for deploying the cannula or needle and delivering fluid through that cannula or needle. Because a wearable drug delivery device (e.g., autoinjector, infuser or microinfuser) has a generally limited ability to increase the pressure at which a fluid is delivered to the patient, a variable increase in the pressure required to pass fluid through the cannula or needle into the tissue may unpredictably reduce the amount of fluid (e.g., drug) delivered to the patient or may require that the device be engineered to provide higher pressures, which pressures may not always be required. Consequently, a systematic approach to reducing the pressure required to deliver fluid through the cannula or needle may not only limit the need to engineer such a delivery device to provide higher pressures, it may ensure that a specific amount of fluid is consistently provided to the patient, improving the reliability of the device.

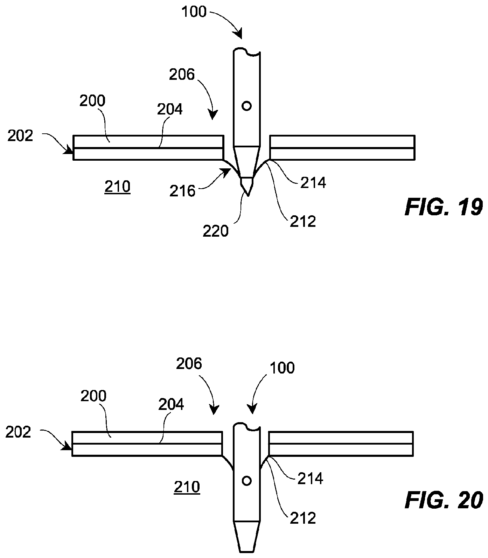

In particular, the resistance to fluid flow in such a wearable device may come about as a consequence of an effect referred to herein as "tenting" As illustrated in Fig. 17, a wearable drug delivery device has a housing 200 with an adhesive layer 202 disposed on an outer surface 204 of the housing 200, which adhesive layer 202 may include a fabric backing 201 and an adhesive 203 according to certain embodiments. See Fig. 17 (while not specifically illustrated in Figs. 19 and 20, the adhesive layer 202 in these Figs. may be defined as illustrated in Fig. 17 as well). In particular, the adhesive layer 202 is disposed on the housing 200 except for a region 206 disposed about the cannula or needle 100 (which, as illustrated, is similar to the embodiment illustrated in Figs. 2-5). This region 206 may be referred to as adhesive-free, and according to those embodiments wherein the adhesive layer 202 includes both a fabric backing 201 and an adhesive 203, the region 206 would be free of both the backing and the adhesive while still termed "adhesive-free."

For example, the region 206 may be adhesive-free in that an opening 208 is formed in the housing 200. According to certain embodiments, the opening 208 may be made as small as is possible while still providing for the free passage of the cannula 100. For example, according to one embodiment, the opening 208 is circular and has a diameter that is not more than twice the diameter of the cannula 100 (by which phrase it is also understood that the opening 208 must be greater than the diameter of the cannula 100 or rigid needle so that the cannula or rigid needle may be disposed through the opening 208 in an operative state of the wearable device and the cannula 100).

While the skin 210 (and associated subcutaneous tissue) to which the drug delivery device is attached has some degree of elasticity (which elasticity may vary from person to person), the adhesive layer 202 disposed about the region 206 attaches the device (and in particular, the housing 200) to the skin surface 212 so as to hold the skin surface 212 at a boundary 214 substantially fixed relative to the housing 200 (see also Fig. 18). While there may be some movement at or along this boundary 214, it is not substantial. As a consequence, when the cannula or needle 100 is automatically advanced or deployed by the action of the drug delivery device in the direction of the skin surface 212, the skin surface 212 starts to stretch relative to the fixed boundary 214. Furthermore, as the cannula or needle 100 passes through the skin 210, the insertion of the cannula 100 causes dragging of the skin tissue 210 because of friction between the cannula 100 and the skin 210. This causes a "tenting" action to occur about the cannula or needle 100 as illustrated in Fig. 19 with reference to numeral 216.

As a brief aside, it will be recognized that the cannula 100 in Figs. 17-20 is illustrated disposed about a structure that is used to insert the cannula into the skin, and which may be removed thereafter to permit fluid to pass through the cannula (compare Figs. 19 and 20). This structure, which may be referred to as an introducer needle 220, need not be present in all embodiments according to the present disclosure. However, according to certain embodiments, the introducer needle 220 is disposed in the cannula 100 and is used to introduce the cannula 100 into the skin 210, after which it may be removed from cannula 100 (see Fig. 20).

As illustrated in Fig. 20, the cannula 100 has now been inserted into the skin 210, and the surface 212 of the skin 210 has generally attempted to return to its original position relative to the outer surface 204 of the drug delivery device housing 200. However, the friction between the cannula 100 and the skin tissue 210 prevents the tissue from completely returning to its initial, or rest, state. As such, the residual forces of the skin and subcutaneous tissues against the tip of the cannula 100 increases the pressure required to deliver fluid through the tip of the cannula 100, and may completely obstruct the tip of the cannula 100 as well.

According to any of the embodiments of the cannula or needle in Figs. 2-15, the pressure required to pass fluid into the tissue may be reduced by, for example, increasing the overall surface area of tissue exposed to the fluid, through the introduction of side ports, etc. In addition, by vibrating the tissue, according to the mechanism of Fig. 16, some of the residual force in the skin tissue 210 may be released, thereby decreasing the pressure and potential for occlusion. The embodiments of Figs. 2-15 may be used in combination with the vibration provided by the embodiment of Fig. 16, or the embodiments of Figs. 2-15 and 16 may be used separately. In any event, it is believed that the embodiments disclosed herein may provide a solution to the tenting of the skin caused at the time the cannula or needle is automatically introduced or deployed into the patient by a wearable drug delivery device. It is also believed that by reducing the size of the opening (fenestration) 208 in the adhesive layer 202, there will be a reduction in tenting and associated residual forces.

As also mentioned above, the reservoir 52 may be filled with a drug or pharmaceutical product. For example, the reservoir may be filled with colony stimulating factors, such as G-CSF. Such G-CSF agents include, but are not limited to, Neupogen® (filgrastim) and Neulasta® (pegfilgrastim).

In various other embodiments, the drug delivery device may be used with various pharmaceutical products, which use may or may not occur under the same conditions as described above for G-CSF. These products may include, for example, an erythropoiesis stimulating agent (ESA), which may be in a liquid or a lyophilized form. An ESA is any molecule that stimulates erythropoiesis, such as Epogen® (epoetin alfa), Aranesp® (darbepoetin alfa), Dynepo® (epoetin delta), Mircera® (methyoxy polyethylene glycol-epoetin beta), Hematide®, MRK-2578, INS-22, Retacrit® (epoetin zeta), Neorecormon® (epoetin beta), Silapo® (epoetin zeta), Binocrit® (epoetin alfa), epoetin alfa Hexal, Abseamed® (epoetin alfa), Ratioepo® (epoetin theta), Eporatio® (epoetin theta), Biopoin® (epoetin theta), epoetin alfa, epoetin beta, epoetin zeta, epoetin theta, and epoetin delta, as well as the molecules or variants or analogs thereof as disclosed in the following patents or patent applications: U.S. Pat. Nos. 4,703,008; 5,441,868; 5,547,933; 5,618,698; 5,621,080; 5,756,349; 5,767,078; 5,773,569; 5,955,422; 5,986,047; 6,583,272; 7,084,245; and 7,271,689; and PCT Publ. Nos. WO 91/05867; WO 95/05465; WO 96/40772; WO 00/24893; WO 01/81405; and WO 2007/136752.

An ESA can be an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, epoetin alfa, epoetin beta, epoetin delta, epoetin omega, epoetin iota, epoetin zeta, and analogs thereof, pegylated erythropoietin, carbamylated erythropoietin, mimetic peptides (including EMP1/hematide), and mimetic antibodies. Exemplary erythropoiesis stimulating proteins include erythropoietin, darbepoetin, erythropoietin agonist variants, and peptides or antibodies that bind and activate erythropoietin receptor (and include compounds reported in U.S. Publ. Nos. 2003/0215444 and 2006/0040858) as well as erythropoietin molecules or variants or analogs thereof as disclosed in the following patents or patent applications,

U.S. Pat. Nos. 4,703,008; 5,441,868; 5,547,933; 5,618,698; 5,621,080; 5,756,349; 5,767,078; 5,773,569; 5,955,422; 5,830,851; 5,856,298; 5,986,047; 6,030,086; 6,310,078; 6,391,633; 6,583,272; 6,586,398; 6,900,292; 6,750,369; 7,030,226; 7,084,245; and 7,217,689; US Publ. Nos. 2002/0155998; 2003/0077753; 2003/0082749; 2003/0143202; 2004/0009902; 2004/0071694; 2004/0091961; 2004/0143857; 2004/0157293; 2004/0175379; 2004/0175824; 2004/0229318; 2004/0248815; 2004/0266690; 2005/0019914; 2005/0026834; 2005/0096461; 2005/0107297; 2005/0107591; 2005/0124045; 2005/0124564; 2005/0137329; 2005/0142642; 2005/0143292; 2005/0153879; 2005/0158822; 2005/0158832; 2005/0170457; 2005/0181359; 2005/0181482; 2005/0192211; 2005/0202538; 2005/0227289; 2005/0244409; 2006/0088906; and 2006/0111279; and PCT Publ. Nos. WO 91/05867; WO 95/05465; WO 99/66054; WO 00/24893; WO 01/81405; WO 00/61637; WO 01/36489; WO 02/014356; WO 02/19963; WO 02/20034; WO 02/49673; WO 02/085940; WO 03/029291; WO 2003/055526; WO 2003/084477; WO 2003/094858; WO 2004/002417; WO 2004/002424; WO 2004/009627; WO 2004/024761; WO 2004/033651; WO 2004/035603; WO 2004/043382; WO 2004/101600; WO 2004/101606; WO 2004/101611; WO 2004/106373; WO 2004/018667; WO 2005/001025; WO 2005/001136; WO 2005/021579; WO 2005/025606; WO 2005/032460; WO 2005/051327; WO 2005/063808; WO 2005/063809; WO 2005/070451; WO 2005/081687; WO 2005/084711; WO 2005/103076; WO 2005/100403; WO 2005/092369; WO 2006/50959; WO 2006/02646; and WO 2006/29094.

Examples of other pharmaceutical products for use with the device may include, but are not limited to, antibodies such as Vectibix® (panitumumab), Xgeva™ (denosumab) and Prolia™ (denosamab); other biological agents such as Enbrel® (etanercept, TNF-receptor /Fc fusion protein, TNF blocker), Neulasta® (pegfilgrastim, pegylated filgastrim, pegylated G-CSF, pegylated hu-Met-G-CSF), Neupogen® (filgrastim , G-CSF, hu-MetG-CSF), and Nplate® (romiplostim); small molecule drugs such as Sensipar® (cinacalcet). The device may also be used with a therapeutic antibody, a polypeptide, a protein or other chemical, such as an iron, for example, ferumoxytol, iron dextrans, ferric glyconate, and iron sucrose. The pharmaceutical product may be in liquid form, or reconstituted from lyophilized form.

Among particular illustrative proteins are the specific proteins set forth below, including fusions, fragments, analogs, variants or derivatives thereof:

OPGL specific antibodies, peptibodies, and related proteins, and the like (also referred to as RANKL specific antibodies, peptibodies and the like), including fully humanized and human OPGL specific antibodies, particularly fully humanized monoclonal antibodies, including but not limited to the antibodies described in PCT Publ. No. WO 03/002713 as to OPGL specific antibodies and antibody related proteins, particularly those having the sequences set forth therein, particularly, but not limited to, those denoted therein: 9H7; 18B2; 2D8; 2E11; 16E1; and 22B3, including the OPGL specific antibodies having either the light chain of SEQ ID NO: 2 as set forth therein in Figure 2 and/or the heavy chain of SEQ ID NO:4, as set forth therein in Figure 4;

Myostatin binding proteins, peptibodies, and related proteins, and the like, including myostatin specific peptibodies, particularly those described in US Publ. No. 2004/0181033 and PCT Publ. No. WO 2004/058988, particularly in parts pertinent to myostatin specific peptibodies, including but not limited to peptibodies of the mTN8-19 family, including those of SEQ ID NOS: 305-351, including TN8-19-1 through TN8-19-40, TN8-19 con1 and TN8-19 con2; peptibodies of the mL2 family of SEQ ID NOS: 357-383; the mL15 family of SEQ ID NOS: 384-409; the mL17 family of SEQ ID NOS: 410-438; the mL20 family of SEQ ID NOS: 439-446; the mL21 family of SEQ ID NOS: 447-452; the mL24 family of SEQ ID NOS: 453-454; and those of SEQ ID NOS: 615-631;

IL-4 receptor specific antibodies, peptibodies, and related proteins, and the like, particularly those that inhibit activities mediated by binding of IL-4 and/or IL-13 to the receptor, including those described in PCT Publ. No. WO 2005/047331 or PCT Appl. No. PCT/US2004/03742 and in US Publ. No. 2005/112694, particularly in parts pertinent to IL-4 receptor specific antibodies, particularly such antibodies as are described therein, particularly, and without limitation, those designated therein: L1H1; L1H2; L1H3; L1H4; L1H5; L1H6; L1H7; L1H8; L1H9; L1H10; L1H11; L2H1; L2H2; L2H3; L2H4; L2H5; L2H6; L2H7; L2H8; L2H9; L2H10; L2H11; L2H12; L2H13; L2H14; L3H1; L4H1; L5H1; L6H1;

Interleukin 1-receptor 1 ("IL1-RF') specific antibodies, peptibodies, and related proteins, and the like, including but not limited to those described in U.S. Publ. No. 2004/097712A1 in parts pertinent to IL1-R1 specific binding proteins, monoclonal antibodies in particular, especially, without limitation, those designated therein: 15CA, 26F5, 27F2, 24E12, and 10H7;

Ang2 specific antibodies, peptibodies, and related proteins, and the like, including but not limited to those described in PCT Publ. No. WO 03/057134 and U.S. Publ No. 2003/0229023, particularly in parts pertinent to Ang2 specific antibodies and peptibodies and the like, especially those of sequences described therein and including but not limited to: L1(N); L1(N) WT; L1(N) 1K WT; 2xL1(N); 2xL1(N) WT; Con4 (N), Con4 (N) 1K WT, 2xCon4 (N) 1K; L1C; L1C 1K; 2xL1C; Con4C; Con4C 1K; 2xCon4C 1K; Con4-L1 (N); Con4-L1C; TN-12-9 (N); C17 (N); TN8-8(N); TN8-14 (N); Con 1 (N), also including anti-Ang 2 antibodies and formulations such as those described in PCT Publ. No. WO 2003/030833 particularly Ab526; Ab528; Ab531; Ab533; Ab535; Ab536; Ab537; Ab540; Ab543; Ab544; Ab545; Ab546; A551; Ab553; Ab555; Ab558; Ab559; Ab565; AbF1AbFD; AbFE; AbFJ; AbFK; AbG1D4; AbGC1E8; AbH1C12; AblA1; AblF; AblK, AblP; and AblP, in their various permutations as described therein;

NGF specific antibodies, peptibodies, and related proteins, and the like including, in particular, but not limited to those described in US Publ. No. 2005/0074821 and US Patent No. 6,91 9,426, particularly as to NGF-specific antibodies and related proteins in this regard, including in particular, but not limited to, the NGF-specific antibodies therein designated 4D4, 4G6, 6H9, 7H2, 14D10 and 14D11;

CD22 specific antibodies, peptibodies, and related proteins, and the like, such as those described in US Patent No. 5,789,554, as to CD22 specific antibodies and related proteins, particularly human CD22 specific antibodies, such as but not limited to humanized and fully human antibodies, including but not limited to humanized and fully human monoclonal antibodies, particularly including but not limited to human CD22 specific IgG antibodies, such as, for instance, a dimer of a human-mouse monoclonal hLL2 gamma-chain disulfide linked to a human-mouse monoclonal hLL2 kappa-chain, including, but limited to, for example, the human CD22 specific fully humanized antibody in Epratuzumab, CAS registry number 501423-23-0;

IGF-1 receptor specific antibodies, peptibodies, and related proteins, and the like, such as those described in PCT Publ. No. WO 06/069202, as to IGF-1 receptor specific antibodies and related proteins, including but not limited to the IGF-1 specific antibodies therein designated L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, L52H52, and IGF-1R-binding fragments and derivatives thereof;

Also among non-limiting examples of anti-IGF-lR antibodies for use in the methods and compositions of the present invention are each and all of those described in:
(i) US Publ. No. 2006/0040358 (published February 23, 2006), 2005/0008642 (published January 13, 2005), 2004/0228859 (published November 18, 2004), including but not limited to, for instance, antibody 1A (DSMZ Deposit No. DSM ACC 2586), antibody 8 (DSMZ Deposit No. DSM ACC 2589), antibody 23 (DSMZ Deposit No. DSM ACC 2588) and antibody 18 as described therein;
(ii) PCT Publ. No. WO 06/138729 (published December 28, 2006) and WO 05/016970 (published February 24, 2005), and Lu et al., 2004, J Biol. Chem. 279:2856-65, including but not limited to antibodies 2F8, A12, and IMC-A12 as described therein;
(iii) PCT Publ. No. WO 07/012614 (published February 1, 2007), WO 07/000328 (published January 4, 2007), WO 06/013472 (published February 9, 2006), WO 05/058967 (published June 30, 2005), and WO 03/059951 (published July 24, 2003);
(iv) US Publ. No. 2005/0084906 (published April 21, 2005), including but not limited to antibody 7C10, chimaeric antibody C7C10, antibody h7C10, antibody 7H2M, chimaeric antibody *7C10, antibody GM 607, humanized antibody 7C10 version 1, humanized antibody 7C10 version 2, humanized antibody 7C10 version 3, and antibody 7H2HM, as described therein;
(v) US Publ. Nos. 2005/0249728 (published November 10, 2005), 2005/0186203 (published August 25, 2005), 2004/0265307 (published December 30, 2004), and 2003/0235582 (published December 25, 2003) and Maloney et al., 2003, Cancer Res. 63:5073-83, including but not limited to antibody EM164, resurfaced EM164, humanized EM164, huEM164 v1.0, huEM164 v1.1, huEM164 v1.2, and huEM164 v1.3 as described therein;
(vi) US Pat. No. 7,037,498 (issued May 2, 2006), US Publ. Nos. 2005/0244408 (published November 30, 2005) and 2004/0086503 (published May 6, 2004), and Cohen, et al., 2005, Clinical Cancer Res. 11:2063-73, e.g., antibody CP-751,871, including but not limited to each of the antibodies produced by the hybridomas having the ATCC accession numbers PTA-2792, PTA-2788, PTA-2790, PTA-2791, PTA-2789, PTA-2793, and antibodies 2.12.1, 2.13.2, 2.14.3, 3.1.1, 4.9.2, and 4.17.3, as described therein;
(vii) US Publ. Nos. 2005/0136063 (published June 23, 2005) and 2004/0018191 (published January 29, 2004), including but not limited to antibody 19D12 and an antibody comprising a heavy chain encoded by a polynucleotide in plasmid 15H12/19D12 HCA (y4), deposited at the ATCC under number PTA-5214, and a light chain encoded by a polynucleotide in plasmid 15H12/19D12 LCF (κ), deposited at the ATCC under number PTA-5220, as described therein; and
(viii) US Publ. No. 2004/0202655 (published October 14, 2004), including but not limited to antibodies PINT-6A1, PINT-7A2, PINT-7A4, PINT-7A5, PINT-7A6, PINT-8A1, PINT-9A2, PINT-11A1, PINT-11A2, PINT-11A3, PINT-11A4, PINT-11A5, PINT-11A7, PINT-11A12, PINT-12A1, PINT-12A2, PINT-12A3, PINT-12A4, and PINT-12A5, as described therein; particularly as to the aforementioned antibodies, peptibodies, and related proteins and the like that target IGF-1 receptors;

B-7 related protein 1 specific antibodies, peptibodies, related proteins and the like ("B7RP-1," also is referred to in the literature as B7H2, ICOSL, B7h, and CD275), particularly B7RP-specific fully human monoclonal IgG2 antibodies, particularly fully human IgG2 monoclonal antibody that binds an epitope in the first immunoglobulin-like domain of B7RP-1, especially those that inhibit the interaction of B7RP-1 with its natural receptor, ICOS, on activated T cells in particular, especially, in all of the foregoing regards, those disclosed in U.S. Publ. No. 2008/0166352 and PCT Publ. No. WO 07/011941, as to such antibodies and related proteins, including but not limited to antibodies designated therein as follow: 16H (having light chain variable and heavy chain variable sequences SEQ ID NO:1 and SEQ ID NO:7 respectively therein); 5D (having light chain variable and heavy chain variable sequences SEQ ID NO:2 and SEQ ID NO:9 respectively therein); 2H (having light chain variable and heavy chain variable sequences SEQ ID NO:3 and SEQ ID NO: 10 respectively therein); 43H (having light chain variable and heavy chain variable sequences SEQ ID NO:6 and SEQ ID NO:14 respectively therein); 41H (having light chain variable and heavy chain variable sequences SEQ ID NO:5 and SEQ ID NO:13 respectively therein); and 15H (having light chain variable and heavy chain variable sequences SEQ ID NO:4 and SEQ ID NO:12 respectively therein),;

IL-15 specific antibodies, peptibodies, and related proteins, and the like, such as, in particular, humanized monoclonal antibodies, particularly antibodies such as those disclosed in U.S. Publ. Nos. 2003/0138421; 2003/023586; and 2004/0071702; and US Patent No. 7,153,507, as to IL-15 specific antibodies and related proteins, including peptibodies, including particularly, for instance, but not limited to, HuMax IL-15 antibodies and related proteins, such as, for instance, 146B7;

IFN gamma specific antibodies, peptibodies, and related proteins and the like, especially human IFN gamma specific antibodies, particularly fully human anti-IFN gamma antibodies, such as, for instance, those described in US Publ. No. 2005/0004353, as to IFN gamma specific antibodies, particularly, for example, the antibodies therein designated 1118; 1118*; 1119; 1121; and 1121*. The entire sequences of the heavy and light chains of each of these antibodies, as well as the sequences of their heavy and light chain variable regions and complementarity determining regions, as disclosed in the foregoing US Publication and in Thakur et al., Mol. Immunol. 36:1107-1115 (1999). In addition, description of the properties of these antibodies provided in the foregoing US publication. Specific antibodies include those having the heavy chain of SEQ ID NO: 17 and the light chain of SEQ ID NO:18; those having the heavy chain variable region of SEQ ID NO:6 and the light chain variable region of SEQ ID NO:8; those having the heavy chain of SEQ ID NO: 19 and the light chain of SEQ ID NO:20; those having the heavy chain variable region of SEQ ID NO:10 and the light chain variable region of SEQ ID NO:12; those having the heavy chain of SEQ ID NO:32 and the light chain of SEQ ID NO:20; those having the heavy chain variable region of SEQ ID NO:30 and the light chain variable region of SEQ ID NO:12; those having the heavy chain sequence of SEQ ID NO:21 and the light chain sequence of SEQ ID NO:22; those having the heavy chain variable region of SEQ ID NO:14 and the light chain variable region of SEQ ID NO:16; those having the heavy chain of SEQ ID NO:21 and the light chain of SEQ ID NO:33; and those having the heavy chain variable region of SEQ ID NO:14 and the light chain variable region of SEQ ID NO:31, as disclosed in the foregoing US Publication. A specific antibody contemplated is antibody 1119 as disclosed in foregoing US Publication and having a complete heavy chain of SEQ ID NO:17 as disclosed therein and having a complete light chain of SEQ ID NO:18 as disclosed therein;

TALL-1 specific antibodies, peptibodies, and the related proteins, and the like, and other TALL specific binding proteins, such as those described in U.S. Publ. Nos. 2003/0195156 and 2006/0135431, as to TALL-1 binding proteins, particularly the molecules of Tables 4 and 5B, as disclosed in the foregoing US Publications;

Parathyroid hormone ("PTH") specific antibodies, peptibodies, and related proteins, and the like, such as those described in US Patent No. 6,756,480, particularly in parts pertinent to proteins that bind PTH;

Thrombopoietin receptor ("TPO-R") specific antibodies, peptibodies, and related proteins, and the like, such as those described in US Patent No. 6,835,809, particularly in parts pertinent to proteins that bind TPO-R;

Hepatocyte growth factor ("HGF") specific antibodies, peptibodies, and related proteins, and the like, including those that target the HGF/SF:cMet axis (HGF/SF:c-Met), such as the fully human monoclonal antibodies that neutralize hepatocyte growth factor/scatter (HGF/SF) described in US Publ. No. 2005/0118643 and PCT Publ. No. WO 2005/017107, huL2G7 described in US Patent No. 7,220,410 and OA-5d5 described in US Patent Nos. 5,686,292 and 6,468,529 and in PCT Publ. No. WO 96/38557, particularly in parts pertinent to proteins that bind HGF;

TRAIL-R2 specific antibodies, peptibodies, related proteins and the like, such as those described in US Patent No. 7,521,048, particularly in parts pertinent to proteins that bind TRAIL-R2;

Activin A specific antibodies, peptibodies, related proteins, and the like, including but not limited to those described in US Publ. No. 2009/0234106, particularly in parts pertinent to proteins that bind Activin A;

TGF-beta specific antibodies, peptibodies, related proteins, and the like, including but not limited to those described in US Patent No. 6,803,453 and US Publ. No. 2007/0110747, particularly in parts pertinent to proteins that bind TGF-beta;

Amyloid-beta protein specific antibodies, peptibodies, related proteins, and the like, including but not limited to those described in PCT Publ. No. WO 2006/081171, particularly in parts pertinent to proteins that bind amyloid-beta proteins. One antibody contemplated is an antibody having a heavy chain variable region comprising SEQ ID NO: 8 and a light chain variable region having SEQ ID NO: 6 as disclosed in the International Publication;

c-Kit specific antibodies, peptibodies, related proteins, and the like, including but not limited to those described in Publ. No. 2007/0253951, particularly in parts pertinent to proteins that bind c-Kit and/or other stem cell factor receptors;

OX40L specific antibodies, peptibodies, related proteins, and the like, including but not limited to those described in US Appl. No. 11/068,289, particularly in parts pertinent to proteins that bind OX40L and/or other ligands of the OXO40 receptor; and

Other exemplary proteins, including Activase® (alteplase, tPA); Aranesp® (darbepoetin alfa); Epogen® (epoetin alfa, or erythropoietin); GLP-1, Avonex® (interferon beta-la); Bexxar® (tositumomab, anti-CD22 monoclonal antibody); Betaseron® (interferon-beta); Campath® (alemtuzumab, anti-CD52 monoclonal antibody); Dynepo® (epoetin delta); Velcade® (bortezomib); MLN0002 (anti- α4β7 mAb); MLN1202 (anti-CCR2 chemokine receptor mAb); Enbrel® (etanercept, TNF-receptor /Fc fusion protein, TNF blocker); Eprex® (epoetin alfa); Erbitux® (cetuximab, anti-EGFR / HER1 / c-ErbB-1); Genotropin® (somatropin, Human Growth Hormone); Herceptin® (trastuzumab, anti-HER2/neu (erbB2) receptor mAb); Humatrope® (somatropin, Human Growth Hormone); Humira® (adalimumab); insulin in solution; Infergen® (interferon alfacon-1); Natrecor® (nesiritide; recombinant human B-type natriuretic peptide (hBNP); Kineret® (anakinra); Leukine® (sargamostim, rhuGM-CSF); LymphoCide® (epratuzumab, anti-CD22 mAb); Benlysta™ (lymphostat B, belimumab, anti-BlyS mAb); Metalyse® (tenecteplase, t-PA analog); Mircera® (methoxy polyethylene glycol-epoetin beta); Mylotarg® (gemtuzumab ozogamicin); Raptiva® (efalizumab); Cimzia® (certolizumab pegol, CDP 870); Soliris™ (eculizumab); pexelizumab (anti-C5 complement); Numax® (MEDI-524); Lucentis® (ranibizumab); Panorex® (17-1A, edrecolomab); Trabio® (lerdelimumab); TheraCim hR3 (nimotuzumab); Omnitarg (pertuzumab, 2C4); Osidem® (IDM-1); OvaRex® (B43.13); Nuvion® (visilizumab); cantuzumab mertansine (huC242-DM1); NeoRecormon® (epoetin beta); Neumega® (oprelvekin, human interleukin-11); Neulasta® (pegylated filgastrim, pegylated G-CSF, pegylated hu-Met-G-CSF); Neupogen® (filgrastim , G-CSF, hu-MetG-CSF); Orthoclone OKT3® (muromonab-CD3, anti-CD3 monoclonal antibody); Procrit® (epoetin alfa); Remicade® (infliximab, anti-TNFa monoclonal antibody); Reopro® (abciximab, anti-GP lIb/Ilia receptor monoclonal antibody); Actemra® (anti-IL6 Receptor mAb); Avastin® (bevacizumab), HuMax-CD4 (zanolimumab); Rituxan® (rituximab, anti-CD20 mAb); Tarceva® (erlotinib); Roferon-A®-(interferon alfa-2a); Simulect® (basiliximab); Prexige® (lumiracoxib); Synagis® (palivizumab); 146B7-CHO (anti-IL15 antibody, see US Patent No. 7,153,507); Tysabri® (natalizumab, anti-a4integrin mAb); Valortim® (MDX-1303, anti-B. anthracis protective antigen mAb); ABthrax™; Vectibix® (panitumumab); Xolair® (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax® (daclizumab); Zenapax® (daclizumab, anti-IL-2Rα mAb); Zevalin® (ibritumomab tiuxetan); Zetia® (ezetimibe); Orencia® (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxinl mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-1103); anti-IGFIR mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP 10 Ulcerative Colitis mAb (MDX-1100); anti-LLY antibody; BMS-66513; anti-Mannose Receptor/hCGβ mAb (MDX-1307); anti-mesothelin dsFv-PE38 conjugate (CAT-5001); anti-PDlmAb (MDX-1106 (ONO-4538)); anti-PDGFRα antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; anti-ZP3 mAb (HuMax-ZP3); NVS Antibody #1; and NVS Antibody #2.

Also included can be a sclerostin antibody, such as but not limited to romosozumab, blosozumab, or BPS 804 (Novartis). Further included can be therapeutics such as rilotumumab, bixalomer, trebananib, ganitumab, conatumumab, motesanib diphosphate, brodalumab, vidupiprant, panitumumab, denosumab, NPLATE, PROLIA, VECTIBIX or XGEVA. Additionally, included in the device can be a monoclonal antibody (IgG) that binds human Proprotein Convertase Subtilisin/Kexin Type 9 (PCSK9), e.g. US 8,030,547, US13/469,032, WO2008/057457, WO2008/057458, WO2008/057459, WO2008/063382, WO2008/133647, WO2009/100297, WO2009/100318, WO2011/037791, WO2011/053759, WO2011/053783, WO2008/125623, WO2011/072263, WO2009/055783, WO2012/0544438, WO2010/029513, WO2011/111007, WO2010/077854, WO2012/088313, WO2012/101251, WO2012/101252, WO2012/101253, WO2012/109530, and WO2001/031007.

Although the preceding text sets forth a detailed description of different embodiments of the invention, it should be understood that the legal scope of the invention is defined by the words of the claims set forth at the end of this patent. The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent, that would still fall within the scope of the claims defining the invention.

## Claims

1. A wearable drug delivery device (50) comprising:
a housing (200);
an adhesive layer (202) disposed on an outer surface of the housing (200) for attaching the drug delivery device (50) to a patient's skin;
a reservoir (52);
a volume of a drug product disposed in the reservoir (52), the drug product comprising a granulocyte colony-stimulating factor (G-CSF);
a blunt cannula (54, 100) having a cylindrical wall (102) that defines an axial passage (104) between a first end (106) and a second end (108) of the cannula (54, 100), the second end (108) of the cannula (54, 100) is connected in fluid communication with the reservoir (52); and
an introducer needle (220) disposed in the cannula (54, 100) adapted for introducing the cannula (54, 100) into the patient's skin and subsequently withdrawing from the cannula (54, 100) prior to delivery of the drug product to the patient, **characterized in that** the cannula (54, 100) has at least one side port (130) formed in the wall (102) of the cannula (54, 100) at the first end (106), and wherein the cannula is initially within the housing and subsequently at least partially deployed from the housing in an operative state.

2. The drug delivery device (50) according to claim 1, further comprising a barrel and a plunger disposed within the barrel to define the reservoir (52).

3. The drug delivery device (50) according to claim 2, further comprising a drive for moving the plunger through the barrel.

4. A wearable drug delivery device (50) comprising:
a housing (200);
an adhesive layer (202) disposed on an outer surface of the housing for attaching the drug delivery device (50) to a patient's skin;
a reservoir (52), wherein a barrel and a plunger disposed within the barrel define the reservoir;
a volume of a drug product disposed in the reservoir (52), the drug product comprising a granulocyte colony-stimulating factor (G-CSF);
a blunt cannula (54, 100) having a cylindrical wall (102) that defines an axial passage (104) between a first end (106) and a second end (108) of the cannula (54, 100), the second end (108) of the cannula (54, 100) configured to be operably connected in fluid communication with the reservoir (52); and
a mechanical drive (56) operably coupled to the reservoir (52) for forcing the drug product from the reservoir through the cannula (54, 100), **characterized in that** the cannula (54, 100) has at least one side port (130) formed in the wall (102) of the cannula (54, 100) at the first end (106), and wherein the cannula is initially within the housing and subsequently at least partially deployed from the housing in an operative state.

5. The drug delivery device (50) of claim 4, further comprising an introducer needle (220) disposed in the cannula (54, 100) adapted for introducing the cannula (54, 100) into the patient's skin and subsequently withdrawing from the cannula (54, 100) prior to delivery of the drug product to the patient.

6. The drug delivery device (50) of claim 1 or 4, wherein the introducer needle (220) extends beyond the first end (106) of the cannula (54, 100) when introducing the cannula (54, 100) into the patient's skin.

7. The drug delivery device according to claim 1 or 4, wherein the at least one side port (130) comprises:
(a) a pair of side ports (130),
(b) a pair of side ports (130) aligned with each other across the axial passage (104),
(c) a circular opening formed in the wall to permit flow transverse to the axial passage (104),
(d) an elongated slit (140) formed in the wall to permit flow transverse to the axial passage,
(e) an elongated slot (140) formed in the wall to permit flow transverse to the axial passage (104), or
(f) at least one side port (130) formed in the first end (106) of the cannula (54, 100) distal from a first tapered region (110).

8. The drug delivery device (50) according to claim 1 or 4, wherein the first end (106) of the cannula (54, 100) includes a first tapered region (110) that comprises at least one bevel (150, 151), further optionally comprising one of:
(a) the first tapered region (110) comprising only one bevel (150, 151),
(b) the first tapered region (110) comprising two bevels intersecting each other (152, 154),
(c) the first tapered region (110) comprising two negative or inverted bevels (156, 158) intersecting each other, or
(d) the first end of the cannula includes a pattern of openings (160) disposed about an opening in the first tapered region (110), wherein optionally, the pattern of openings (160) comprises a pattern of recesses into the wall.

9. The drug delivery device (50) according to claim 1 or 4, wherein the cannula (54, 100) has at least one external recessed region (170) recessed toward the axial passage relative to adjoining surface regions.

10. The drug delivery device (50) according to claim 9, wherein the at least one external recessed region (170) is defined by (a) a pattern of ribs (172), or (b) a pattern of grooves (176).

## Patentansprüche

1. Tragbare Arzneimittelabgabevorrichtung (50), umfassend:
ein Gehäuse (200),
eine adhäsive Schicht (202), die auf einer äußeren Oberfläche des Gehäuses (200) angeordnet ist, um die Arzneimittelabgabevorrichtung (50) an der Haut eines Patienten zu befestigen,
ein Reservoir (52),
ein Volumen eines Arzneimittelprodukts, das in dem Reservoir (52) angeordnet ist,
wobei das Arzneimittelprodukt einen Granulozytenkolonie-stimulierenden Faktor (G-CSF) umfasst,
eine stumpfe Kanüle (54, 100) mit einer zylindrischen Wand (102), die einen axialen Durchgang (104) zwischen einem ersten Ende (106) und einem zweiten Ende (108) der Kanüle (54, 100) definiert, wobei das zweite Ende (108) der Kanüle (54, 100) in Fluidverbindung mit dem Reservoir (52) verbunden ist, und
eine in der Kanüle (54, 100) angeordnete Einführungsnadel (220), die zum Einführen der Kanüle (54, 100) in die Haut des Patienten und zum anschließenden Herausziehen aus der Kanüle (54, 100) vor der Abgabe des Arzneimittelprodukts an den Patienten eingerichtet ist, **dadurch gekennzeichnet, dass** die Kanüle (54, 100) mindestens eine Seitenöffnung (130) aufweist, die in der Wand (102) der Kanüle (54, 100) an dem ersten Ende (106) ausgebildet ist, und wobei sich die Kanüle anfänglich innerhalb des Gehäuses befindet und anschließend in einem Funktionszustand zumindest teilweise aus dem Gehäuse ausgefahren ist.

2. Arzneimittelabgabevorrichtung (50) gemäß Anspruch 1, ferner einen Zylinder und einen innerhalb des Zylinders angeordneten Kolben umfassend, um das Reservoir (52) zu definieren.

3. Arzneimittelabgabevorrichtung (50) gemäß Anspruch 2, ferner einen Antrieb zum Bewegen des Kolbens durch den Zylinder umfassend.

4. Tragbare Arzneimittelabgabevorrichtung (50), umfassend:
ein Gehäuse (200),
eine adhäsive Schicht (202), die auf einer äußeren Oberfläche des Gehäuses (200) angeordnet ist, um die Arzneimittelabgabevorrichtung (50) an der Haut eines Patienten zu befestigen,
ein Reservoir (52), wobei ein Zylinder und ein innerhalb des Zylinders angeordneter Kolben das Reservoir definieren,
ein Volumen eines Arzneimittelprodukts, das in dem Reservoir (52) angeordnet ist,
wobei das Arzneimittelprodukt einen Granulozytenkolonie-stimulierenden Faktor (G-CSF) umfasst,
eine stumpfe Kanüle (54, 100) mit einer zylindrischen Wand (102), die einen axialen Durchgang (104) zwischen einem ersten Ende (106) und einem zweiten Ende (108) der Kanüle (54, 100) definiert, wobei das zweite Ende (108) der Kanüle (54, 100) so konfiguriert ist, dass es betriebsfähig in Fluidverbindung mit dem Reservoir (52) verbunden ist, und
einen mechanischen Antrieb (56), der betriebsfähig mit dem Reservoir (52) gekoppelt ist, um das Arzneimittelprodukt aus dem Reservoir durch die Kanüle (54, 100) zu drücken, **dadurch gekennzeichnet, dass** die Kanüle (54, 100) mindestens eine seitliche Öffnung (130) aufweist, welche in der Wand (102) der Kanüle (54, 100) an dem ersten Ende (106) ausgebildet ist, und wobei die Kanüle sich anfänglich innerhalb des Gehäuses befindet und anschließend in einem Funktionszustand mindestens teilweise aus dem Gehäuse ausgefahren ist.

5. Arzneimittelabgabevorrichtung (50) nach Anspruch 4, ferner eine in der Kanüle (54, 100) angeordnete Einführungsnadel (220) umfassend, die zum Einführen der Kanüle (54, 100) in die Haut des Patienten und zum anschließenden Herausziehen aus der Kanüle (54, 100) vor der Abgabe des Arzneimittelprodukts an den Patienten eingerichtet ist.

6. Arzneimittelabgabevorrichtung (50) nach Anspruch 1 oder 4, wobei sich die Einführungsnadel (220) über das erste Ende (106) der Kanüle (54, 100) hinaus erstreckt, wenn die Kanüle (54, 100) in die Haut des Patienten eingeführt wird.

7. Arzneimittelabgabevorrichtung gemäß Anspruch 1 oder 4, wobei die mindestens eine Seitenöffnung (130) umfasst:
(a) ein Paar von Seitenöffnungen (130),
(b) ein Paar von Seitenöffnungen (130), die miteinander über den axialen Durchgang (104) ausgerichtet sind,
(c) eine kreisförmige Öffnung, die in der Wand ausgebildet ist, um eine Strömung quer zum axialen Durchgang (104) zu ermöglichen,
(d) einen länglichen Schlitz (140), der in der Wand ausgebildet ist, um eine Strömung quer zum axialen Durchgang zu ermöglichen,
(e) eine längliche Aussparung (140), die in der Wand ausgebildet ist, um eine Strömung quer zum axialen Durchgang (104) zu ermöglichen, oder
(f) mindestens eine Seitenöffnung (130), die im ersten Ende (106) der Kanüle (54, 100) distal von einem ersten verjüngten Bereich (110) ausgebildet ist.

8. Arzneimittelabgabevorrichtung (50) gemäß Anspruch 1 oder 4, wobei das erste Ende (106) der Kanüle (54, 100) einen ersten verjüngten Bereich (110) beinhaltet, welcher mindestens eine Abschrägung (150, 151) umfasst, ferner wahlweise eines von:
(a) dem ersten verjüngten Bereich (110), der nur eine Abschrägung (150, 151) umfasst,
(b) dem ersten verjüngten Bereich (110), der zwei einander schneidende Abschrägungen (152, 154) umfasst,
(c) dem ersten verjüngten Bereich (110), der zwei einander schneidende negative oder umgekehrte Abschrägungen (156, 158) umfasst, umfassend oder
(d) das erste Ende der Kanüle weist ein Muster von Öffnungen (160) auf, die um eine Öffnung in dem ersten verjüngten Bereich (110) herum angeordnet sind, wobei das Muster von Öffnungen (160) wahlweise ein Muster von Aussparungen in der Wand umfasst.

9. Arzneimittelabgabevorrichtung (50) gemäß Anspruch 1 oder 4, wobei die Kanüle (54, 100) mindestens einen äußeren vertieften Bereich (170) aufweist, der in Richtung des axialen Durchgangs relativ zu angrenzenden Oberflächenbereichen vertieft ist.

10. Arzneimittelabgabevorrichtung (50) gemäß Anspruch 9, wobei der mindestens eine äußere ausgesparte Bereich (170) definiert ist durch (a) ein Muster von Rippen (172) oder (b) ein Muster von Rillen (176).

## Revendications

1. Dispositif portable d'administration de médicament (50) comprenant :
un logement (200) ;
une couche adhésive (202) disposée sur une surface externe du logement (200) pour la fixation du dispositif d'administration de médicament (50) à la peau d'un patient ;
un réservoir (52) ;
un volume d'un produit médicamenteux disposé dans le réservoir (52), le produit médicamenteux comprenant un facteur de stimulation de colonie de granulocytes (G-CSF) ;
une canule (54, 100) à extrémité franche ayant une paroi cylindrique (102) qui définit un passage axial (104) entre une première extrémité (106) et une seconde extrémité (108) de la canule (54, 100), la seconde extrémité (108) de la canule (54, 100) étant reliée par communication fluidique au réservoir (52) ; et
une aiguille d'introduction (220) disposée dans la canule (54, 100) adaptée pour introduire la canule (54, 100) dans la peau du patient et par la suite pour retirer la canule (54, 100) avant l'administration du produit médicamenteux au patient, **caractérisée en ce que** la canule (54, 100) présente au moins un orifice latéral (130) formé dans la paroi (102) de la canule (54, 100) au niveau de la première extrémité (106), et la canule se situant initialement à l'intérieur du logement et par la suite au moins partiellement déployée du logement sous un état de fonctionnement.

2. Dispositif d'administration de médicament (50) selon la revendication 1, comprenant en outre un cylindre et un piston disposé à l'intérieur du cylindre pour définir le réservoir (52).

3. Dispositif d'administration de médicament (50) selon la revendication 2, comprenant en outre un dispositif d'entraînement pour déplacer le piston à travers le cylindre.

4. Dispositif portable d'administration de médicament (50) comprenant :
un logement (200) ;
une couche adhésive (202) disposée sur une surface externe du logement pour la fixation du dispositif d'administration de médicament (50) à la peau d'un patient ;
un réservoir (52), dans lequel un cylindre et un piston disposé à l'intérieur du cylindre définissent le réservoir ;
un volume d'un produit médicamenteux disposé dans le réservoir (52), le produit médicamenteux comprenant un facteur de stimulation de colonie de granulocytes (G-CSF) ;
une canule (54, 100) à extrémité franche ayant une paroi cylindrique (102) qui définit un passage axial (104) entre une première extrémité (106) et une seconde extrémité (108) de la canule (54, 100), la seconde extrémité (108) de la canule (54, 100) conçue pour être fonctionnellement reliée en communication fluidique avec le réservoir (52) ; et
un dispositif d'entraînement mécanique (56) fonctionnellement relié au réservoir (52) pour forcer le produit médicamenteux depuis le réservoir à travers la canule (54, 100), **caractérisé en ce que** la canule (54, 100) présente au moins un orifice latéral (130) formé dans la paroi (102) de la canule (54, 100) au niveau de la première extrémité (106), et la canule se situant initialement à l'intérieur du logement et par la suite au moins partiellement déployée depuis le logement sous un état de fonctionnement.

5. Dispositif d'administration de médicament (50) selon la revendication 4, comprenant en outre une aiguille d'introduction (220) disposée dans la canule (54, 100) adaptée pour l'introduction de la canule (54, 100) dans la peau du patient et par la suite pour le retrait de la canule (54, 100) avant l'administration du produit médicamenteux au patient.

6. Dispositif d'administration de médicament (50) selon la revendication 1 ou 4, l'aiguille d'introduction (220) s'étendant au-delà de la première extrémité (106) de la canule (54, 100) lors de l'introduction de la canule (54, 100) dans la peau du patient.

7. Dispositif d'administration de médicament selon la revendication 1 ou 4, ledit au moins un orifice latéral (130) comprenant :
(a) une paire d'orifices latéraux (130),
(b) une paire d'orifices latéraux (130) alignés l'un par rapport à l'autre à travers le passage axial (104),
(c) une ouverture circulaire formée dans la paroi pour permettre le flux transversal vers le passage axial (104),
(d) une fente allongée (140) formée dans la paroi pour permettre le flux transversal vers le passage axial,
(e) une fente allongée (140) formée dans la paroi pour permettre le flux transversal vers le passage axial (104), ou
(f) au moins un orifice latéral (130) formé dans la première extrémité (106) de la canule (54, 100) distale depuis une première région effilée (110).

8. Dispositif d'administration de médicament (50) selon la revendication 1 ou 4, la première extrémité (106) de la canule (54, 100) comprenant une première région effilée (110) qui comprend au moins un biseau (150, 151), comprenant éventuellement en outre l'une parmi :
(a) la première région effilée (110) comprenant uniquement un biseau (150, 151),
(b) la première région effilée (110) comprenant deux biseaux en intersection l'un avec l'autre (152, 154),
(c) la première région effilée (110) comprenant deux biseaux négatifs ou inversés entrant en intersection l'un avec l'autre (156, 158), ou
(d) la première extrémité de la canule comprenant un profil d'ouvertures (160) disposé autour d'une ouverture dans la première région effilée (110), dans laquelle éventuellement, le profil des ouvertures (160) comprend un profil des renfoncements dans la paroi.

9. Dispositif d'administration de médicament (50) selon la revendication 1 ou 4, la canule (54, 100) ayant au moins une région renfoncée externe (170) renfoncée vers le passage axial par rapport aux régions de surface adjacentes.

10. Dispositif d'administration de médicament (50) selon la revendication 9, la au moins une région renfoncée externe (170) étant définie par (a) un profil de nervures (172), ou (b) un profil de rainures (176).
